# EUROPEAN PATENT SPECIFICATION

(11) **EP 4 046 568 B1**
(45) Date of publication and mention of the grant of the patent: **20.03.2024**
(21) Application number: 21157655.8
(22) Date of filing: 17.02.2021
(51) Int. Cl.: A61B 5/00, A61B 5/021, A61B 5/0245, A61B 5/0535, A61B 5/08, A61B 5/113, A61B 5/145, A61B 5/33, A61B 5/346, A61N 1/39

(54) **CARDIO PULMONARY RESUSCITATION FEEDBACK SYSTEM**
RÜCKKOPPLUNGSSYSTEM FÜR KARDIO-PULMONALE WIEDERBELEBUNG
SYSTÈME DE RÉTROACTION DE RÉANIMATION CARDIOPULMONAIRE

(43) Date of publication of application: 24.08.2022
(73) Proprietor: Stryker European Operations Limited, Carrigtwohill, Co. Cork T45 HX08 (IE)
(72) Inventor: DONAGHY, Dymphna, Belfast, BT3 9ED (GB); ANDERSON, Johnny, Belfast, BT3 9ED (GB); HARVEY, Adam, Belfast, BT3 9ED (GB); MCALISTER, Olibhear, Belfast, BT3 9ED (GB)
(74) Representative: HGF

(56) References cited:
- US-A1- 2017 065 484
- US-A1- 2017 120 063

## Description

The invention relates to a cardio pulmonary resuscitation (CPR) feedback system, using electrocardiogram (ECG) signals and biosignals to assess the CPR and provide CPR feedback to a person during their performance of CPR on a subject.

The CPR feedback system is used to treat a subject by instructing a person using the system to commence and maintain high quality CPR. CPR involves the compression of the chest of the subject to cause the heart to pump blood around the circulatory system, primarily to provide oxygenated blood to the subject's heart and brain. If the compression of the chest is too shallow, too slow or too fast, then the heart will not pump sufficient oxygenated blood. Changes in chest compression rate and chest compression depth evoke changes in biosignals measured from the subject, thus, these biosignals may be used to guide the person on CPR performance. It is advantageous if CPR feedback is provided to the person during CPR, as this can improve the CPR performance of the person.

US 2017/0065484 discloses a method for determining an efficacy of cardiopulmonary resuscitation (CPR) which includes receiving a plethysmography signal from an oximetry sensor and an electrocardiogram (ECG) signal from an ECG sensor at a processor associated with a patient monitor, the oximetry sensor, or the ECG sensor. The method includes determining a first indicator related to the efficacy of CPR based on the plethysmography signal, using the processor. The method also includes determining a second indicator related to the efficacy of CPR based on the ECG signal, using the processor. The method further includes combining the first indicator and the second indicator to determine a combination metric indicative of the efficacy of CPR, using the processor.

US 2017/0120063 discloses a system for managing treatment for an emergency cardiac event. The system includes memory, one or more electronic ports for receiving ECG signals, and a treatment module executable on one or more processing devices. The module is configured to perform a number of transformations on portions of an ECG signal into frequency domain data, obtain one or more previous values derived from one or more time segments of the ECG, and determine, based on the frequency domain data, a first value and a second value, and determine a probability of therapeutic success. The module is further configured to cause one or more output devices to present an indication of the probability oftherapeutic success.

According to a first aspect of the invention there is provided a cardio pulmonary resuscitation (CPR) feedback system for assessing CPR carried out by a person on a subject and providing CPR feedback to the person, comprising:
an electrocardiogram (ECG) system configured to measure ECG signals of the subject,
a biosignal system configured to measure biosignals of the subject,
a CPR assessment system connected to the ECG system to receive ECG signals of the subject and connected to the biosignal system to receive biosignals of the subject, and
a CPR feedback unit connected to the CPR assessment system and configured to receive CPR feedback signals and issue CPR feedback to the person,
wherein the CPR assessment system is configured to perform the steps:
   (i) establish a reference ECG signal metric using ECG signals of the subject measured prior to commencement of CPR and establish a target biosignal metric,
   (ii) produce a CPR feedback signal advising the person to start CPR,
   (iii) receive ECG signals of the subject measured during a plurality of chest compressions and use the ECG signals to establish a current ECG signal metric of the subject,
   (iv) receive biosignals of the subject measured during the plurality of chest compressions and use the biosignals to establish a current biosignal metric of the subject,
   (v) compare the current ECG signal metric with the reference ECG signal metric and compare the current biosignal metric with the target biosignal metric,
   (vi) when the current ECG signal metric is less than the reference ECG signal metric and the current biosignal metric is less than the target biosignal metric, produce a CPR feedback signal advising the person to improve CPR performance,
   (vii) when the current ECG signal metric is less than the reference ECG signal metric and the current biosignal metric is equal to or greater than the target biosignal metric, increase the target biosignal metric and produce a CPR feedback signal advising the person to improve CPR performance,
   (viii) when the current ECG signal metric is equal to or greater than the reference ECG signal metric, set the reference ECG signal metric equal to the current ECG signal metric and produce a CPR feedback signal advising the person to maintain current CPR performance,
wherein the reference ECG signal metric of the subject and the current ECG signal metric of the subject comprise a score derived from any of one or more time-domain features of ECG signals of the subject, one or more frequency-domain features of ECG signals of the subject, one or more time-domain features and one or more frequency-domain features of ECG signals of the subject,
wherein the target biosignal metric of the subject and the current biosignal metric of the subject comprise at least one biosignal metric component for at least one type of biosignal comprising any of at least one frequency biosignal metric component, at least one amplitude biosignal metric component, at least one frequency biosignal metric component and at least one amplitude biosignal metric component, and
wherein producing a CPR feedback signal advising the person to improve CPR performance comprises any of producing a CPR feedback signal advising the person to push harder, producing a CPR feedback signal advising the person to push faster, producing a CPR feedback signal advising the person to push slower.

In the invention, the CPR feedback system provides dynamic CPR feedback to the person performing CPR on the subject, the CPR feedback being based on repeated monitoring of the chest compressions performed by the person on the subject using biosignal measurements and repeated monitoring of the ECG of the subject.

The biosignal system may be configured to measure one or more type of biosignals of the subject. The types of biosignals may comprise any of chest impedance signals, end-tidal carbon dioxide signals, saturation of peripheral oxygen signals, blood pressure signals, chest compression depth signals. The biosignal system may comprise any of an impedance signal measurement system, a capnograph, an oximeter, a blood pressure measurement system, an accelerometer.

Establishing the reference ECG signal metric may comprise receiving ECG signals from the ECG system measured over a predefined period of time prior to commencement of CPR and using the ECG signals to establish the reference ECG signal metric.

Using the ECG signals to establish the reference ECG signal metric may comprise establishing a score of the ECG signals. The score may relate to quality of the measured ECG signals. The score may be derived from one or more time-domain features of the ECG signals. The time-domain features may comprise any of mean amplitude, peak amplitude, median slope of the ECG signals. The score may be derived from one or more frequency-domain features of the ECG signals. The frequency-domain features may comprise any of amplitude spectrum area (AMSA), power spectrum analysis features, centroid frequency. The score may be derived from one or more time-domain features and one or more frequency-domain features. The time-domain features and the frequency-domain features may be used as measures to estimate a condition of the subject.

Establishing the target biosignal metric may comprise receiving a pre-determined target biosignal metric. The pre-determined target biosignal metric may comprise at least one target biosignal metric component for one or more types of biosignal. The at least one target biosignal metric component for the one or more types of biosignal may comprise any of at least one target frequency biosignal metric component, at least one target amplitude biosignal metric component, at least one target frequency biosignal metric component and at least one target amplitude biosignal metric component.

Establishing the target biosignal metric may comprise (i) receiving one or more types of biosignals measured during a plurality of chest compressions by the person, and (ii) using the or each or some of the types of biosignals to establish the target biosignal metric. The target biosignal metric may comprise at least one target biosignal metric component for the or each or some of the types of biosignal. The at least one target biosignal metric component for the or each or some of the types of biosignal may comprise any of at least one target frequency biosignal metric component, at least one target amplitude biosignal metric component at least one target frequency biosignal metric component and at least one target amplitude biosignal metric component. The target biosignal metric component for each type of biosignals may provide a measure of target CPR performance for the person during CPR chest compressions.

The target amplitude biosignal metric components may comprise any of average amplitude, average local maxima, average local minima, ratio of local maxima to local minima. The target frequency biosignal metric component may comprise dominant frequency.

Receiving ECG signals measured during a plurality of chest compressions may comprise receiving ECG signals measured over a window of predefined duration. The predefined duration may be in a range of approximately 5 seconds to approximately 30 seconds.

Using the ECG signals to establish a current ECG signal metric may comprise establishing a score of the ECG signals. The score may relate to quality of the measured ECG signals. The score may be derived from one or more time-domain feature of the ECG signals. The time-domain features may comprise any of mean amplitude, peak amplitude, median slope of the ECG signals. The score may be derived from one or more frequency-domain features of the ECG signals. The frequency-domain features may comprise any of amplitude spectrum area (AMSA), power spectrum analysis features, centroid frequency. The score may be derived from one or more time-domain features and one or more frequency-domain features. The time-domain features and the frequency-domain features may be used as measures to estimate subject condition.

The ECG signals may be assessed to confirm the presence of ventricular fibrillation (VF) signals.

Receiving biosignals measured during the plurality of chest compressions may comprise receiving biosignals measured over a window of predefined duration. The predefined duration may be in a range of approximately 5 seconds to approximately 30 seconds.

Receiving biosignals measured during the plurality of chest compressions may comprise receiving at least one type of biosignals measured during the plurality of chest compressions. Using the biosignals measured during the plurality of chest compressions to establish the current biosignal metric may comprise establishing at least one current biosignal metric component for at least one type of biosignals. The at least one current biosignal metric component for the at least one type of biosignals may comprise any of at least one current frequency biosignal metric component, at least one current amplitude biosignal metric component, at least one current frequency biosignal metric component and at least one current amplitude biosignal metric component. The current biosignal metric component for the least one type of biosignals may provide a measure of actual CPR performance by the person during the plurality of CPR chest compressions.

The current amplitude biosignal metric components may comprise any of average amplitude, average local maxima, average local minima, ratio of local maxima to local minima. The current frequency biosignal metric component may comprise dominant frequency.

Comparing the current biosignal metric with the target biosignal metric may comprise comparing at least one current biosignal metric component for at least one type of biosignals with at least one equivalent target biosignal metric component for the at least one type of biosignals.

Increasing the target biosignal metric may comprise increasing the metric by 1% to 50%.

The CPR feedback signal advising the person on how to adjust CPR may be received by the feedback unit and cause the feedback unit to issue CPR feedback to the person in the form of 'Push Harder' and/or 'Push Faster'. The CPR feedback signal advising the person to maintain current CPR performance may be received by the feedback unit and cause the feedback unit to issue CPR feedback to the person in the form of 'Good Compressions'.

The CPR assessment system may be configured to repeat steps (iii) to (viii) over a predetermined period of time. The pre-determined period of time may be 2 minutes. When the predetermined period of time has been reached, the CPR assessment system may be configured to produce a CPR feedback signal advising the person to stop CPR. The CPR assessment system may then be configured to repeat steps (i) to (viii).

The CPR assessment system may be further configured to carry out the steps:
produce a CPR feedback signal advising the person to perform CPR chest compressions at a rate equal to a rate of an audible metronome signal emitted by a metronome of the CPR assessment system,
receive ECG signals measured during the chest compressions and use the ECG signals to establish a current ECG signal metric,
compare the current ECG signal metric with the reference ECG signal metric,
when the current ECG signal metric is less than the reference ECG signal metric, adjust the rate of the audible metronome signal, produce a CPR feedback signal advising the person to adjust the rate of performance of the CPR chest compressions to equal the adjusted rate of the audible metronome signal and go back to the second step,
when the current ECG signal metric is equal to or greater than the reference ECG signal metric, produce a CPR feedback signal advising the person to maintain the rate of performance of the CPR chest compressions.

The CPR feedback signal advising the person to adjust the rate of performance of the CPR chest compressions to equal the adjusted rate of the audible metronome signal may be received by the feedback unit and cause the feedback unit to issue CPR feedback to the person in the form of 'Push Faster' or 'Push Slower'. The CPR feedback signal advising the person to maintain the rate of performance of the CPR chest compressions may be received by the feedback unit and cause the feedback unit to issue CPR feedback to the person in the form of 'Push Harder' or 'Good Compressions'.

The ECG system may be configured to measure ECG signals of the subject using signals received from one or more electrodes placed on the subject.

The biosignal system may be configured to measure biosignals of the subject using signals received from one or more sensors placed on the subject.

The CPR feedback system may be a stand-alone system. The CPR feedback system may be part of a further system. The further system may be a defibrillator.

When the CPR feedback system is part of a further system, a CPR start signal may be received from the further system. When the CPR feedback system is part of a further system, a CPR stop signal may be received from the further system.

According to a second aspect of the invention there is provided a defibrillator comprising a CPR feedback system according to the first aspect of the invention.

An embodiment of the invention will now be described, by way of example only, with reference to the following drawings, in which :
Figure 1 is a schematic representation of a CPR feedback system according to the invention, and
Figure 2 is a flowchart of steps carried out by a CPR assessment system of the CPR feedback system of Figure 1.

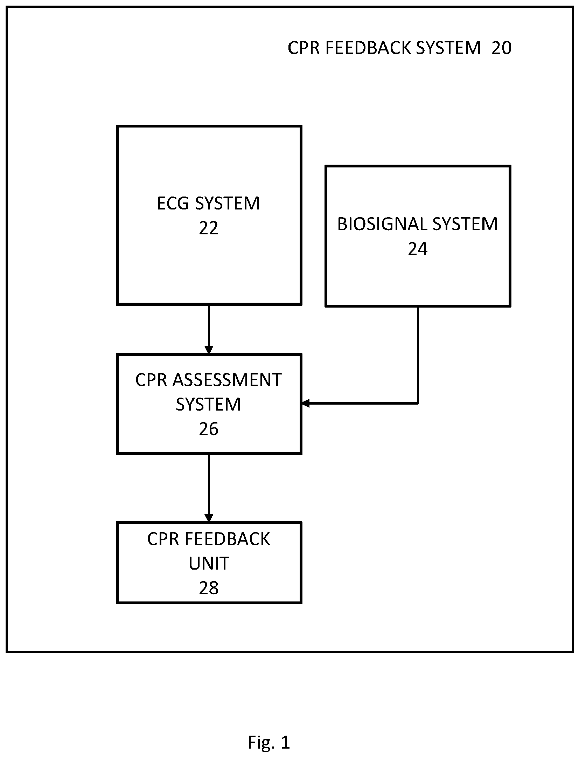

Referring to Figure 1, the CPR feedback system 20 comprises an electrocardiogram (ECG) system 22, a biosignal system 24, a CPR assessment system 26 and a feedback unit 28. The CPR feedback system 20 assesses CPR carried out by a person (not shown) on a subject (not shown) and provides CPR feedback to the person. Th biosignal system 24 comprises each of an impedance signal measurement system, a capnograph, an oximeter, a blood pressure measurement system, an accelerometer.

It will be appreciated that the CPR feedback system 20 will comprise other elements such as an activation mechanism, an ECG processing system, a power source and a sensing unit which is adapted to be attached to the subject.

The ECG system 22 is connected to the sensing unit, which in this embodiment comprises thoracic electrodes, and is configured to measure ECG signals of the subject. The CPR feedback system 20 comprises an algorithm which uses the ECG signals to determine when the subject is exhibiting VF.

The impedance signal measurement system of the biosignal system 24 is connected to the sensing unit, which in this embodiment comprises thoracic electrodes, and measures impedance signals of the subject during chest compressions by the person. The impedance signals of the subject comprise thoracic impedance signals. The impedance signals comprises sinusoidal waves, caused by the CPR chest compressions. The capnograph, oximeter, blood pressure measurement system and accelerometer of the biosignal system 24 are connected to sensors placed on the subject and receive biosignals of the subject from the sensors.

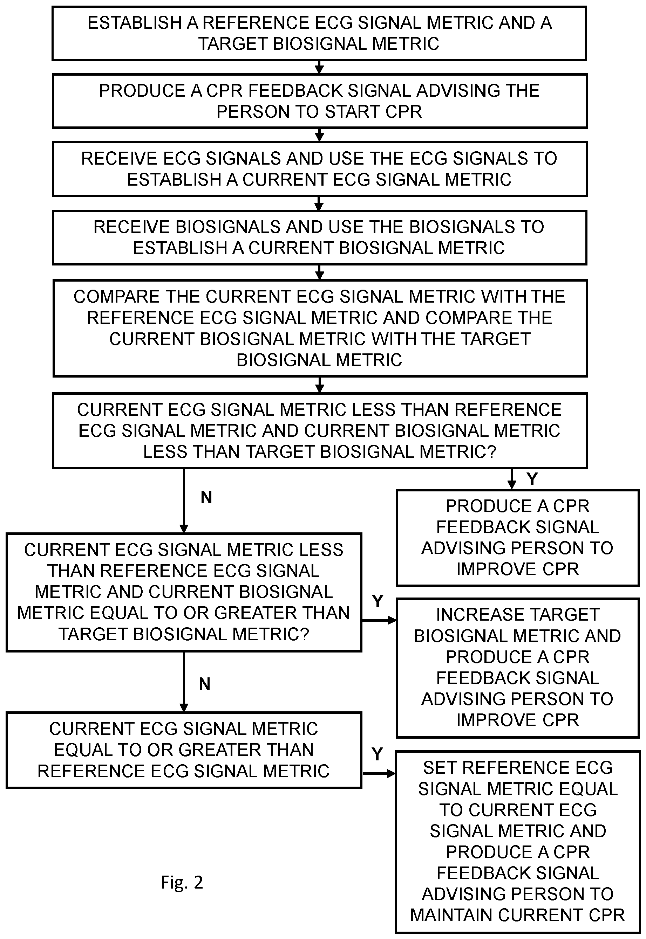

The CPR assessment system 26 is connected to the ECG system 22 and the biosignal system 24. The CPR assessment system 26 receives ECG signals and biosignals and assesses CPR performance by the person on the subject over multiple pluralities of chest compressions, delivered to the subject by the person in a pre-determined time. This comprises performance of a number of steps, described below with reference to Figure 2.

During assessment of the CPR performance of the person, the CPR assessment system 26 produces various feedback signals. The feedback unit 28 is connected to the CPR assessment system 26 and is configured to receive the feedback signals and issue CPR feedback to the person.

Referring to Figure 2, a first embodiment of the steps performed by the CPR assessment system 26 of the CPR feedback system 20 of Figure 1 will be described. In this embodiment, the biosignal system 24 uses its impedance measurement system to measure impedance signals of the subject. The CPR assessment system 26 receives one type of biosignals, i.e. impedance signals.

The CPR assessment system 26 first establishes a reference ECG metric. The CPR assessment system 26 receives ECG signals from the ECG system 22 measured over a predefined period of time of, for example, 5 to 30 seconds prior to commencement of CPR. The ECG signals are used to establish the reference ECG signal metric. This comprises using the ECG signals to establish a reference ECG score of the ECG signals. The reference ECG score relates to quality of the measured ECG signals. In this embodiment, the reference ECG score is derived from one or more time-domain feature of the ECG signals and one or more frequency-domain features of the ECG signals. The time-domain features comprise any of mean amplitude, peak amplitude, median slope of the ECG signals. The frequency-domain features comprise any of amplitude spectrum area (AMSA), power spectrum analysis features, centroid frequency. The time-domain features and the frequency-domain features may be used as measures to estimate subject condition.

The CPR assessment system 26 then establishes a target biosignal metric. In this embodiment, establishing the target biosignal metric comprises producing a CPR feedback signal advising the person to start CPR, receiving biosignals, comprising impedance signals, measured during a plurality of chest compressions by the person, producing a CPR feedback signal advising the person to stop CPR and using the biosignals to establish the target biosignal metric. In this embodiment, the target biosignal metric comprises one target biosignal metric component for the type of biosignals measured, i.e. impedance signals. The target biosignal metric component comprises a target amplitude biosignal metric component of average amplitude of peaks in the impedance signals measured during the plurality of CPR chest compressions by the person. It will be appreciated that the target biosignal metric may comprise more than one target biosignal metric component and that the components may be target amplitude biosignal metric components and/or target frequency biosignal metric components.

The CPR assessment system 26 then produces a CPR feedback signal advising the person to start CPR. This is sent to the feedback unit 28 which issues CPR feedback in the form of a 'Start CPR and Push Hard' instruction to the person using the CPR feedback system 20.

During a plurality of chest compressions by the person, the CPR assessment system 26 receives ECG signals from the ECG system 22, measured over a window of approximately 5 seconds to approximately 30 seconds. The CPR assessment system 26 uses the ECG signals to establish a current ECG signal metric, by establishing a current ECG score of the ECG signals. The current ECG score relates to quality of the measured ECG signals. The current ECG score is derived from one or more time-domain feature of the ECG signals and one or more frequency-domain features. The time-domain features comprise any of mean amplitude, peak amplitude, median slope of the ECG signals. The frequency-domain features comprise any of amplitude spectrum area (AMSA), power spectrum analysis features, centroid frequency. The time-domain features and the frequency-domain features may be used as measures to estimate subject condition.

During the plurality of chest compressions carried out by the person on the subject, the CPR assessment system 26 also receives impedance signals from the impedance measurement system of the biosignal system 24. The impedance signals are measured over the same window of approximately 5 seconds to approximately 30 seconds. The CPR assessment system 26 uses the impedance signals to establish a current biosignal metric. In this embodiment, the current biosignal metric comprises one current biosignal metric component for the impedance signals. The current biosignal metric component comprises a current amplitude biosignal metric component of average amplitude of peaks in the impedance signals measured during the window.

The CPR assessment system 26 compares the current ECG signal metric with the reference ECG signal metric and compares the current impedance signal metric with the target impedance signal metric. This comprises comparing the reference ECG score with the current ECG score and comparing the target amplitude biosignal metric component of average amplitude of impedance signal peaks with the current amplitude biosignal metric component of average amplitude of impedance signal peaks. When the current ECG signal metric is less than the reference ECG signal metric (which suggests that the subject's condition is deteriorating) and the current biosignal metric is less than the target biosignal metric (which suggests that the CPR chest compressions are suboptimal), the CPR assessment system 26 produces a CPR feedback signal advising the person on how to adjust CPR. The feedback signal is sent to the feedback unit 28 which issues CPR feedback in the form of a 'Push Harder' instruction to the person using the CPR feedback system 20.

When the current ECG signal metric is less than the reference ECG signal metric (again suggesting that the subject's condition is deteriorating) and the current biosignal metric is equal to or greater than the target biosignal metric (which suggests that the target impedance signal metric may be improved), the CPR assessment system 26 increases the target biosignal metric and produces a CPR feedback signal advising the person to improve CPR performance. The feedback signal is sent to the feedback unit 28 which issues CPR feedback in the form of a 'Push Harder' instruction to the person using the CPR feedback system 20.

When the current ECG signal metric is equal to or greater than the reference ECG signal metric (suggesting that the subject's condition is improving), the CPR assessment system sets the reference ECG signal metric equal to the current ECG signal metric and produces a CPR feedback signal advising the person to maintain current CPR performance. The feedback signal is sent to the feedback unit 28 which issues CPR feedback in the form of a 'Good Compressions' instruction to the person using the CPR feedback system 20.

In this embodiment, the CPR assessment system 26 is configured to repeat some of the steps described above over a predetermined period of time. The pre-determined period of time is 2 minutes and is measured by a clock of the CPR assessment system 26. When the clock has not reached the pre-determined time the CPR assessment system 26 returns to the step of receiving ECG signals measured during a further plurality of chest compressions by the person. The further plurality of chest compressions do not overlap with the previous plurality of chest compressions. The analysis of the ECG and the biosignals comprising impedance signals and feedback to the subject therefore continues over several pluralities of chest compressions during the 2 min time. When the clock reaches the pre-determined time, the CPR assessment system 26 produces a CPR feedback signal advising the person to stop CPR. The feedback signal is sent to the feedback unit 28 which issues CPR feedback in the form of a 'Stop CPR' instruction to the person using the CPR feedback system 20.

In this embodiment, the CPR assessment system 26 then returns to the steps of establishing a reference ECG signal metric and a target biosignal metric and repeats the further steps as described above. This continues for as long as is deemed necessary.

Referring to Figure 2, a second embodiment of the steps performed by the CPR assessment system 26 of the CPR feedback system 20 of Figure 1 will be described. In this embodiment, the biosignal system 24 uses its oximeter and accelerometer to measure saturation of peripheral oxygen signals and CPR chest compression depth signals of the subject. The CPR assessment system 26 receives two types of biosignals, i.e. oxygen signals and CPR chest compression depth signals.

The CPR assessment system 26 first establishes a reference ECG metric. The CPR assessment system 26 receives ECG signals from the ECG system 22 measured over a predefined period of time of, for example, 5 to 30 seconds prior to commencement of CPR. The ECG signals are used to establish the reference ECG signal metric. This comprises using the ECG signals to establish a reference ECG score of the ECG signals. The reference ECG score relates to quality of the measured ECG signals. In this embodiment, the reference ECG score is derived from one or more time-domain feature of the ECG signals and one or more frequency-domain features of the ECG signals. The time-domain features comprise any of mean amplitude, peak amplitude, median slope of the ECG signals. The frequency-domain features comprise any of amplitude spectrum area (AMSA), power spectrum analysis features, centroid frequency. The time-domain features and the frequency-domain features may be used as measures to estimate subject condition.

The CPR assessment system 26 then establishes a target biosignal metric. In this embodiment, establishing the target biosignal metric comprises receiving a pre-determined target biosignal metric. The pre-determined target biosignal metric comprises at least one target biosignal metric component for each type of biosignals measured. In this embodiment, the target biosignal metric components comprise a target amplitude biosignal metric component of average amplitude of oxygen signals and a target amplitude biosignal metric component of average CPR chest compression depth signals. It will be appreciated that the target biosignal metric may comprise more than one target biosignal metric component and that the components may be target amplitude biosignal metric components and/or target frequency biosignal metric components.

The CPR assessment system 26 then produces a CPR feedback signal advising the person to start CPR. This is sent to the feedback unit 28 which issues CPR feedback in the form of a 'Start CPR and Push Hard' instruction to the person using the CPR feedback system 20.

During a plurality of chest compressions by the person, the CPR assessment system 26 receives ECG signals from the ECG system 22, measured over a window of approximately 5 seconds to approximately 30 seconds. The CPR assessment system 26 uses the ECG signals to establish a current ECG signal metric, by establishing a current ECG score of the ECG signals. The current ECG score relates to quality of the measured ECG signals. The current ECG score is derived from one or more time-domain feature of the ECG signals and one or more frequency-domain features. The time-domain features comprise any of mean amplitude, peak amplitude, median slope of the ECG signals. The frequency-domain features comprise any of amplitude spectrum area (AMSA), power spectrum analysis features, centroid frequency. The time-domain features and the frequency-domain features may be used as measures to estimate subject condition.

During the plurality of chest compressions carried out by the person on the subject, the CPR assessment system 26 also receives oxygen signals from the oximeter of the biosignal system 24 and CPR chest compression depth signals from the accelerometer of the biosignal system 24. The oxygen signals and the CPR chest compression depth signals are measured over the same window of approximately 5 seconds to approximately 30 seconds. The CPR assessment system 26 uses the oxygen signals and the CPR chest compression depth signals to establish a current biosignal metric. In this embodiment, the current biosignal metric comprises a current amplitude biosignal metric component for the oxygen signals of an average amplitude of the oxygen signals and a current amplitude biosignal metric component for the CPR chest compression depth signals of an average CPR chest compression depth.

The CPR assessment system 26 compares the current ECG signal metric with the reference ECG signal metric. This comprises comparing the reference ECG score with the current ECG score. The CPR assessment system 26 further compares the current signal metric with the target signal metric. This comprises comparing the target amplitude biosignal metric component of the oxygen signals with the current amplitude biosignal metric component of the oxygen signals and comparing the target amplitude biosignal metric component of the CPR chest compression depth signals with the current amplitude biosignal metric component of the CPR chest compression depth signals.

When the current ECG signal metric is less than the reference ECG signal metric (which suggests that the subject's condition is deteriorating) and the current biosignal metric is less than the target biosignal metric (which suggests that the CPR chest compressions are suboptimal), the CPR assessment system 26 produces a CPR feedback signal advising the person to improve CPR performance. The feedback signal is sent to the feedback unit 28 which issues CPR feedback in the form of a 'Push Faster', 'Push Slower' or 'Push Harder' instruction to the person using the CPR feedback system 20.

When the current ECG signal metric is less than the reference ECG signal metric (again suggesting that the subject's condition is deteriorating) and the current biosignal metric is equal to or greater than the target biosignal metric (which suggests that the target biosignal metric may be improved), the CPR assessment system 26 increases the target biosignal metric and produces a CPR feedback signal advising the person to improve CPR performance. The feedback signal is sent to the feedback unit 28 which issues CPR feedback in the form of a 'Push Harder' instruction to the person using the CPR feedback system 20.

When the current ECG signal metric is equal to or greater than the reference ECG signal metric (suggesting that the subject's condition is improving), the CPR assessment system sets the reference ECG signal metric equal to the current ECG signal metric and produces a CPR feedback signal advising the person to maintain current CPR performance. The feedback signal is sent to the feedback unit 28 which issues CPR feedback in the form of a 'Good Compressions' instruction to the person using the CPR feedback system 20.

In this embodiment, the CPR assessment system 26 is configured to repeat some of the steps described above over a predetermined period of time. The pre-determined period of time is 2 minutes and is measured by a clock of the CPR assessment system 26. When the clock has not reached the pre-determined time the CPR assessment system 26 returns to the step of receiving ECG signals measured during a further plurality of chest compressions by the person. The further plurality of chest compressions do not overlap with the previous plurality of chest compressions. The analysis of the ECG and the biosignals, comprising oxygen signals and CPR chest compression depth signals, and feedback to the subject therefore continues over several pluralities of chest compressions during the 2 min time. When the clock reaches the pre-determined time, the CPR assessment system 26 produces a CPR feedback signal advising the person to stop CPR. The feedback signal is sent to the feedback unit 28 which issues CPR feedback in the form of a 'Stop CPR' instruction to the person using the CPR feedback system 20.

In this embodiment, the CPR assessment system 26 then returns to the step of establishing a reference ECG signal metric and a target biosignal metric and repeats the further steps as described above. This continues for as long as is deemed necessary.

Thus, the CPR feedback system 20 provides dynamic CPR feedback to the person performing multiple sets of CPR chest compressions on the subject, the CPR feedback being based on monitoring of the sets of chest compressions using biosignals comprising impedance signals, oxygen signals and CPR chest compression depth signals and monitoring of the ECG of the subject. It will be appreciated that other types of biosignals may be used.

In these embodiments, the CPR feedback system 20 is described as a stand-alone device. It will be appreciated that the CPR feedback system 20 may comprise a part of a further system, such as a defibrillator.

## Claims

1. A cardio pulmonary resuscitation (CPR) feedback system (20) for assessing CPR carried out by a person on a subject and providing CPR feedback to the person, comprising:
an electrocardiogram (ECG) system (22) configured to measure ECG signals of the subject,
a biosignal system (24) configured to measure biosignals of the subject,
a CPR assessment system (26) connected to the ECG system (22) to receive ECG signals of the subject and connected to the biosignal system (24) to receive biosignals of the subject, and
a feedback unit (28) connected to the CPR assessment system (26) and configured to receive CPR feedback signals and issue CPR feedback to the person,
wherein the CPR assessment system (26) is configured to perform the steps:
(i) establish a reference ECG signal metric using ECG signals of the subject measured prior to commencement of CPR and establish a target biosignal metric,
(ii) produce a CPR feedback signal advising the person to start CPR,
(iii) receive ECG signals of the subject measured during a plurality of chest compressions and use the ECG signals to establish a current ECG signal metric of the subject,
(iv) receive biosignals of the subject measured during the plurality of chest compressions and use the biosignals to establish a current biosignal metric of the subject,
(v) compare the current ECG signal metric with the reference ECG signal metric and compare the current biosignal metric with the target biosignal metric,
(vi) when the current ECG signal metric is less than the reference ECG signal metric and the current biosignal metric is less than the target biosignal metric, produce a CPR feedback signal advising the person to improve CPR performance,
(vii) when the current ECG signal metric is less than the reference ECG signal metric and the current biosignal metric is equal to or greater than the target biosignal metric, increase the target biosignal metric and produce a CPR feedback signal advising the person to improve CPR performance,
(viii) when the current ECG signal metric is equal to or greater than the reference ECG signal metric, set the reference ECG signal metric equal to the current ECG signal metric and produce a CPR feedback signal advising the person to maintain current CPR performance,
wherein the reference ECG signal metric of the subject and the current ECG signal metric of the subject comprise a score derived from any of one or more time-domain features of ECG signals of the subject, one or more frequency-domain features of ECG signals of the subject, one or more time-domain features and one or more frequency-domain features of ECG signals of the subject,
wherein the target biosignal metric of the subject and the current biosignal metric of the subject comprise at least one biosignal metric component for at least one type of biosignal comprising any of at least one frequency biosignal metric component, at least one amplitude biosignal metric component, at least one frequency biosignal metric component and at least one amplitude biosignal metric component, and
wherein producing a CPR feedback signal advising the person to improve CPR performance comprises any of producing a CPR feedback signal advising the person to push harder, producing a CPR feedback signal advising the person to push faster, producing a CPR feedback signal advising the person to push slower.

2. A CPR feedback system (20) according to claim 1 in which the biosignal system (24) is configured to measure one or more type of biosignals of the subject.

3. A CPR feedback system (20) according to claim 2 in which the types of biosignals comprise any of chest impedance signals, end-tidal carbon dioxide signals, saturation of peripheral oxygen signals, blood pressure signals, chest compression depth signals.

4. A CPR feedback system (20) according to any preceding claim in which establishing the reference ECG signal metric comprises receiving ECG signals of the subject from the ECG system (22) measured over a predefined period of time prior to commencement of CPR and using the ECG signals of the subject to establish the reference ECG signal metric.

5. A CPR feedback system (20) according to any preceding claim in which establishing the target biosignal metric comprises receiving a pre-determined target biosignal metric comprising at least one target biosignal metric component for one or more types of biosignal comprising any of at least one target frequency biosignal metric component, at least one target amplitude biosignal metric component, at least one target frequency biosignal metric component and at least one target amplitude biosignal metric component.

6. A CPR feedback system (20) according to any of claims 1 to 4 in which establishing the target biosignal metric comprises (i) receiving one or more types of biosignals of the subject measured during a plurality of chest compressions by the person, and (ii) using the or each or some of the types of biosignals of the subject to establish the target biosignal metric.

7. A CPR feedback system (20) according to claim 6 in which the target biosignal metric comprises at least one target biosignal metric component for the or each or some of the types of biosignals of the subject comprising any of at least one target frequency biosignal metric component, at least one target amplitude biosignal metric component at least one target frequency biosignal metric component and at least one target amplitude biosignal metric component.

8. A CPR feedback system (20) according to any preceding claim in which using the biosignals of the subject measured during the plurality of chest compressions to establish the current biosignal metric comprises establishing at least one current biosignal metric component for at least one type of biosignals comprising any of at least one current frequency biosignal metric component, at least one current amplitude biosignal metric component, at least one current frequency biosignal metric component and at least one current amplitude biosignal metric component.

9. A CPR feedback system (20) according to claim 8 in which comparing the current biosignal metric with the target biosignal metric comprises comparing at least one current biosignal metric component for the at least one type of biosignals with at least one equivalent target biosignal metric component for the at least one type of biosignals.

10. A CPR assessment system (20) according to any preceding claim configured to repeat steps (iii) to (viii) over a predetermined period of time and when the predetermined period of time has been reached, produce a CPR feedback signal advising the person to stop CPR.

11. A CPR assessment system (20) according to any preceding claim configured to repeat steps (i) to (viii).

12. A CPR assessment system (20) according to any preceding claim configured to carry out the steps:
produce a CPR feedback signal advising the person to perform CPR chest compressions at a rate equal to a rate of an audible metronome signal emitted by a metronome of the CPR assessment system (20),
receive ECG signals measured during the chest compressions and use the ECG signals to establish a current ECG signal metric,
compare the current ECG signal metric with the reference ECG signal metric,
when the current ECG signal metric is less than the reference ECG signal metric, adjust the rate of the audible metronome signal, produce a CPR feedback signal advising the person to adjust the rate of performance of the CPR chest compressions to equal the adjusted rate of the audible metronome signal and go back to the second step,
when the current ECG signal metric is equal to or greater than the reference ECG signal metric, produce a CPR feedback signal advising the person to maintain the rate of performance of the CPR chest compressions.

13. A CPR feedback system (20) according to any preceding claim which is part of a defibrillator.

## Patentansprüche

1. Rückmeldungssystem (20) für kardiopulmonale Wiederbelebung (CPR) zum Bewerten einer CPR, die durch eine Person an einem Subjekt ausgeführt wird, und zum Bereitstellen einer CPR-Rückmeldung an die Person, umfassend:
ein Elektrokardiogramm(EKG)-System (22), das dazu konfiguriert ist, EKG-Signale des Subjekts zu messen,
ein Biosignalsystem (24), das dazu konfiguriert ist, Biosignale des Subjekts zu messen,
ein CPR-Bewertungssystem (26), das mit dem EKG-System (22) verbunden ist, um EKG-Signale des Subjekts zu empfangen, und mit dem Biosignalsystem (24) verbunden ist, um Biosignale des Subjekts zu empfangen, und
eine Rückmeldungseinheit (28), die mit dem CPR-Bewertungssystem (26) verbunden und dazu konfiguriert ist, CPR-Rückmeldungssignale zu empfangen und eine CPR-Rückmeldung an die Person auszugeben,
wobei das CPR-Bewertungssystem (26) dazu konfiguriert ist, die folgenden Schritte durchzuführen:
(i) Erstellen einer Referenz-EKG-Signalmetrik unter Verwendung von EKG-Signalen des Subjekts, die vor Beginn der CPR gemessen wurden, und Erstellen einer Ziel-Biosignalmetrik,
(ii) Erzeugen eines CPR-Rückmeldungssignals, das der Person empfiehlt, die CPR zu starten,
(iii) Empfangen von EKG-Signalen des Subjekts, die während einer Vielzahl von Thoraxkompressionen gemessen werden, und Verwenden der EKG-Signale, um eine aktuelle EKG-Signalmetrik des Subjekts zu erstellen,
(iv) Empfangen von Biosignalen des Subjekts, die während der Vielzahl von Thoraxkompressionen gemessen werden, und Verwenden der Biosignale, um eine aktuelle Biosignalmetrik des Subjekts zu erstellen,
(v) Vergleichen der aktuellen EKG-Signalmetrik mit der Referenz-EKG-Signalmetrik und Vergleichen der aktuellen Biosignalmetrik mit der Ziel-Biosignalmetrik,
(vi) wenn die aktuelle EKG-Signalmetrik geringer ist als die Referenz-EKG-Signalmetrik und die aktuelle Biosignalmetrik geringer ist als die Ziel-Biosignalmetrik, Erzeugen eines CPR-Rückmeldungssignals, das der Person empfiehlt, die CPR-Durchführung zu verbessern,
(vii) wenn die aktuelle EKG-Signalmetrik geringer ist als die Referenz-EKG-Signalmetrik und die aktuelle Biosignalmetrik gleich oder stärker als die Ziel-Biosignalmetrik ist, Erhöhen der Ziel-Biosignalmetrik und Erzeugen eines CPR-Rückmeldungssignals, das der Person empfiehlt, die CPR-Durchführung zu verbessern,
(viii) wenn die aktuelle EKG-Signalmetrik gleich oder stärker als die Referenz-EKG-Signalmetrik ist, Einstellen der Referenz-EKG-Signalmetrik gleich der aktuellen EKG-Signalmetrik und Erzeugen eines CPR-Rückmeldungssignals, das der Person empfiehlt, die aktuelle CPR-Durchführung beizubehalten,
wobei die Referenz-EKG-Signalmetrik des Subjekts und die aktuelle EKG-Signalmetrik des Subjekts einen Punktwert umfassen, der aus einem von einem oder mehreren Zeitbereichsmerkmalen von EKG-Signalen des Subjekts, einem oder mehreren Frequenzbereichsmerkmalen von EKG-Signalen des Subjekts, einem oder mehreren Zeitbereichsmerkmalen und einem oder mehreren Frequenzbereichsmerkmalen von EKG-Signalen des Subjekts abgeleitet ist,
wobei die Ziel-Biosignalmetrik des Subjekts und die aktuelle Biosignalmetrik des Subjekts mindestens eine Biosignalmetrikkomponente für mindestens eine Art von Biosignal umfassen, die eine von mindestens einer Frequenz-Biosignalmetrikkomponente, mindestens einer Amplituden-Biosignalmetrikkomponente, mindestens einer Frequenz-Biosignalmetrikkomponente und mindestens einer Amplituden-Biosignalmetrikkomponente umfasst, und
wobei das Erzeugen eines CPR-Rückmeldungssignals, das der Person empfiehlt, die CPR-Durchführung zu verbessern, eines von Erzeugen eines CPR-Rückmeldungssignals, das der Person empfiehlt, stärker zu drücken, Erzeugen eines CPR-Rückmeldungssignals, das der Person empfiehlt, schneller zu drücken, und Erzeugen eines CPR-Rückmeldungssignals, das der Person empfiehlt, langsamer zu drücken, umfasst.

2. CPR-Rückmeldungssystem (20) nach Anspruch 1, bei dem das Biosignalsystem (24) dazu konfiguriert ist, eine oder mehrere Art von Biosignalen des Subjekts zu messen.

3. CPR-Rückmeldungssystem (20) nach Anspruch 2, bei dem die Arten von Biosignalen eines von Thoraximpedanzsignalen, endexspiratorischen Kohlendioxidsignalen, peripheren Sauerstoffsättigungssignalen, Blutdrucksignalen und Thoraxkompressionstiefensignalen umfassen.

4. CPR-Rückmeldungssystem (20) nach einem vorhergehenden Anspruch, bei dem das Erstellen der Referenz-EKG-Signalmetrik Empfangen von EKG-Signalen des Subjekts von dem EKG-System (22), die über einen vordefinierten Zeitraum vor Beginn der CPR gemessen wurden, und Verwenden der EKG-Signale des Subjekts, um die Referenz-EKG-Signalmetrik zu erstellen, umfasst.

5. CPR-Rückmeldungssystem (20) nach einem vorhergehenden Anspruch, bei dem das Erstellen der Ziel-Biosignalmetrik Empfangen einer vorbestimmten Ziel-Biosignalmetrik umfasst, die mindestens eine Ziel-Biosignalmetrikkomponente für eine oder mehrere Arten von Biosignal umfasst, die eine von mindestens einer Zielfrequenz-Biosignalmetrikkomponente, mindestens einer Zielamplituden-Biosignalmetrikkomponente, mindestens einer Zielfrequenz-Biosignalmetrikkomponente und mindestens einer Zielamplituden-Biosignalmetrikkomponente umfassen.

6. CPR-Rückmeldungssystem (20) nach einem der Ansprüche 1 bis 4, bei dem das Erstellen der Ziel-Biosignalmetrik (i) Empfangen einer oder mehrerer Arten von Biosignalen des Subjekts, die während einer Vielzahl von Thoraxkompressionen durch die Person gemessen werden, und (ii) Verwenden der oder jeder oder einiger der Arten von Biosignalen des Subjekts, um die Ziel-Biosignalmetrik zu erstellen, umfasst.

7. CPR-Rückmeldungssystem (20) nach Anspruch 6, bei dem die Ziel-Biosignalmetrik mindestens eine Ziel-Biosignalmetrikkomponente für die oder jede oder einige der Arten von Biosignalen des Subjekts umfasst, die eine von mindestens einer Zielfrequenz-Biosignalmetrikkomponente, mindestens einer Zielamplituden-Biosignalmetrikkomponente, mindestens einer Zielfrequenz-Biosignalmetrikkomponente und mindestens einer Zielamplituden-Biosignalmetrikkomponente umfassen.

8. CPR-Rückmeldungssystem (20) nach einem vorhergehenden Anspruch, bei dem das Verwenden der Biosignale des Subjekts, die während der Vielzahl von Thoraxkompressionen gemessen werden, um die aktuelle Biosignalmetrik zu erstellen, Erstellen mindestens einer aktuellen Biosignalmetrikkomponente für mindestens eine Art von Biosignalen umfasst, die eine von mindestens einer aktuellen Frequenz-Biosignalmetrikkomponente, mindestens einer aktuellen Amplituden-Biosignalmetrikkomponente, mindestens einer aktuellen Frequenz-Biosignalmetrikkomponente und mindestens einer aktuellen Amplituden-Biosignalmetrikkomponente umfasst.

9. CPR-Rückmeldungssystem (20) nach Anspruch 8, bei dem das Vergleichen der aktuellen Biosignalmetrik mit der Ziel-Biosignalmetrik Vergleichen mindestens einer aktuellen Biosignalmetrikkomponente für die mindestens eine Art von Biosignalen mit mindestens einer äquivalenten Ziel-Biosignalmetrikkomponente für die mindestens eine Art von Biosignalen umfasst.

10. CPR-Bewertungssystem (20) nach einem vorhergehenden Anspruch, das dazu konfiguriert ist, die Schritte (iii) bis (viii) über einen vorbestimmten Zeitraum zu wiederholen und, wenn der vorbestimmte Zeitraum erreicht wurde, ein CPR-Rückmeldungssignal zu erzeugen, das der Person empfiehlt, die CPR zu beenden.

11. CPR-Bewertungssystem (20) nach einem vorhergehenden Anspruch, das dazu konfiguriert ist, die Schritte (i) bis (viii) zu wiederholen.

12. CPR-Bewertungssystem (20) nach einem vorhergehenden Anspruch, das dazu konfiguriert ist, die folgenden Schritte auszuführen:
Erzeugen eines CPR-Rückmeldungssignals, das der Person empfiehlt, CPR-Thoraxkompressionen mit einer Geschwindigkeit durchzuführen, die einer Geschwindigkeit eines hörbaren Metronomsignals entspricht, das durch ein Metronom des CPR-Bewertungssystems (20) ausgesendet wird,
Empfangen von EKG-Signalen, die während der Thoraxkompressionen gemessen werden, und Verwenden der EKG-Signale, um eine aktuelle EKG-Signalmetrik zu erstellen,
Vergleichen der aktuellen EKG-Signalmetrik mit der Referenz-EKG-Signalmetrik,
wenn die aktuelle EKG-Signalmetrik geringer als die Referenz-EKG-Signalmetrik ist, Anpassen der Geschwindigkeit des hörbaren Metronomsignals, Erzeugen eines CPR-Rückmeldungssignals, das der Person empfiehlt, die Durchführungsgeschwindigkeit der CPR-Thoraxkompressionen anzupassen, so dass sie der angepassten Geschwindigkeit des hörbaren Metronomsignals entspricht, und Zurückkehren zum zweiten Schritt,
wenn die aktuelle EKG-Signalmetrik gleich oder stärker als die Referenz-EKG-Signalmetrik ist, Erzeugen eines CPR-Rückmeldungssignals, das der Person empfiehlt, die Durchführungsgeschwindigkeit der CPR-Thoraxkompressionen beizubehalten.

13. CPR-Rückmeldungssystem (20) nach einem vorhergehenden Anspruch, das Teil eines Defibrillators ist.

## Revendications

1. Système de rétroaction de réanimation cardio-pulmonaire (RCP) (20) permettant d'évaluer la RCP effectuée par une personne sur un sujet et de fournir une rétroaction de RCP à la personne, comprenant :
un système d'électrocardiogramme (ECG) (22) configuré pour mesurer les signaux d'ECG du sujet,
un système de biosignaux (24) configuré pour mesurer les biosignaux du sujet,
un système d'évaluation de RCP (26) branché au système d'ECG (22) de manière à recevoir les signaux d'ECG du sujet et branché au système de biosignaux (24) de manière à recevoir les biosignaux du sujet, et
une unité de rétroaction (28) branchée au système d'évaluation de RCP (26) et configurée pour recevoir les signaux de rétroaction de RCP et émettre une rétroaction de RCP à la personne,
ledit système d'évaluation de RCP (26) étant configuré pour effectuer les étapes :
(i) établissement d'une mesure de signal d'ECG de référence à l'aide des signaux d'ECG du sujet mesurés avant le début de la RCP et établissement d'une mesure de biosignal cible,
(ii) production d'un signal de rétroaction de RCP conseillant à la personne de commencer la RCP,
(iii) réception de signaux d'ECG du sujet mesurés pendant une pluralité de compressions thoraciques et utilisation des signaux d'ECG pour établir une mesure de signal d'ECG courante du sujet,
(iv) réception de biosignaux du sujet mesurés pendant la pluralité de compressions thoraciques et utilisation des biosignaux pour établir une mesure de biosignal courante du sujet,
(v) comparaison de la mesure de signal d'ECG courante avec la mesure de signal d'ECG de référence et comparaison de la mesure de biosignal courante avec la mesure de biosignal cible,
(vi) lorsque la mesure de signal d'ECG courante est inférieure à la mesure de signal d'ECG de référence et que la mesure de biosignal courante est inférieure à la mesure de biosignal cible, production d'un signal de rétroaction de RCP conseillant à la personne d'améliorer l'exécution de la RCP,
(vii) lorsque la mesure de signal d'ECG courante est inférieure à la mesure de signal d'ECG de référence et que la mesure de biosignal courante est supérieure ou égale à la mesure de biosignal cible, augmentation de la mesure de biosignal cible et production d'un signal de rétroaction de RCP conseillant à la personne d'améliorer l'exécution de la RCP,
(viii) lorsque la mesure de signal d'ECG courante est supérieure ou égale à la mesure de signal d'ECG de référence, réglage de la mesure de signal d'ECG de référence égale à la mesure de signal d'ECG courante et production d'un signal de rétroaction de RCP conseillant à la personne de maintenir l'exécution de la RCP courante,
ladite mesure de signal ECG de référence du sujet et ladite mesure de signal d'ECG courante du sujet comprenant un score dérivé de l'une ou plusieurs caractéristiques de domaine temporel des signaux d'ECG du sujet, l'une ou plusieurs caractéristiques de domaine fréquentiel des signaux d'ECG du sujet, l'une ou plusieurs caractéristiques de domaine temporel et l'une ou plusieurs caractéristiques de domaine fréquentiel des signaux d'ECG du sujet,
ladite mesure de biosignal cible du sujet et ladite mesure de biosignal courante du sujet comprenant au moins une composante de mesure de biosignal pour au moins un type de biosignal comprenant l'une quelconque composante parmi au moins une composante de mesure de biosignal de fréquence, au moins une composante de mesure de biosignal d'amplitude, au moins une composante de mesure de biosignal de fréquence et au moins une composante de mesure de biosignal d'amplitude, et
ladite production d'un signal de rétroaction de RCP conseillant à la personne d'améliorer l'exécution de la RCP comprenant l'une quelconque production parmi la production d'un signal de rétroaction de RCP conseillant à la personne de pousser plus fort, la production d'un signal de rétroaction de RCP conseillant à la personne de pousser plus vite, et la production d'un signal de rétroaction de RCP conseillant à la personne de pousser plus lentement.

2. Système de rétroaction de RCP (20) selon la revendication 1, ledit système de biosignaux (24) étant configuré pour mesurer un ou plusieurs type de biosignaux du sujet.

3. Système de rétroaction de RCP (20) selon la revendication 2, lesdits types de biosignaux comprenant l'un quelconque signal parmi les signaux d'impédance thoracique, les signaux de dioxyde de carbone de fin d'expiration, les signaux de saturation en oxygène périphérique, les signaux de pression artérielle, les signaux de profondeur de compression thoracique.

4. Système de rétroaction de RCP (20) selon l'une quelconque des revendications précédentes, ledit établissement de la mesure de signal d'ECG de référence comprenant la réception de signaux d'ECG du sujet provenant du système d'ECG (22) mesurés sur une période de temps prédéfinie avant le début de la RCP et l'utilisation des signaux d'ECG du sujet pour établir la mesure de signal d'ECG de référence.

5. Système de rétroaction de RCP (20) selon l'une quelconque des revendications précédentes, ledit établissement de la mesure de biosignal cible comprenant la réception d'une mesure de biosignal cible prédéfinie comprenant au moins une composante de mesure de biosignal cible pour un ou plusieurs types de biosignal comprenant l'une quelconque composante parmi au moins une composante de mesure de biosignal de fréquence cible, au moins une composante de mesure de biosignal d'amplitude cible, au moins une composante de mesure de biosignal de fréquence cible et au moins une composante de mesure de biosignal d'amplitude cible.

6. Système de rétroaction de RCP (20) selon l'une quelconque des revendications 1 à 4, ledit établissement de la mesure de biosignal cible comprenant (i) la réception d'un ou plusieurs types de biosignaux du sujet mesurés pendant une pluralité de compressions thoraciques par la personne, et (ii) l'utilisation des ou de chacun ou de certains des types de biosignaux du sujet pour établir la mesure de biosignal cible.

7. Système de rétroaction de RCP (20) selon la revendication 6, ladite mesure de biosignal cible comprenant au moins une composante de mesure de biosignal cible pour les ou chacun ou certains des types de biosignaux du sujet comprenant l'une quelconque composante parmi au moins une composante de mesure de biosignal de fréquence cible, au moins une composante de mesure de biosignal d'amplitude cible, au moins une composante de mesure de biosignal de fréquence cible et au moins une composante de mesure de biosignal d'amplitude cible.

8. Système de rétroaction de RCP (20) selon l'une quelconque des revendications précédentes, ladite utilisation des biosignaux du sujet mesurés pendant la pluralité de compressions thoraciques pour établir la mesure de biosignal courante comprenant l'établissement d'au moins une composante de mesure de biosignal courante pour au moins un type de biosignaux comprenant une quelconque composante parmi au moins une composante de mesure de biosignal de fréquence courante, au moins une composante de mesure de biosignal d'amplitude courante, au moins une composante de mesure de biosignal de fréquence courante et au moins une composante de mesure de biosignal d'amplitude courante.

9. Système de rétroaction de RCP (20) selon la revendication 8, ladite comparaison de la mesure de biosignal courante avec la mesure de biosignal cible comprenant la comparaison d'au moins une composante de mesure de biosignal courante pour ledit au moins un type de biosignaux avec au moins une composante de mesure de biosignal cible équivalente pour ledit au moins un type de biosignaux.

10. Système d'évaluation de RCP (20) selon l'une quelconque des revendications précédentes, configuré pour répéter les étapes (iii) à (viii) sur une période de temps prédéfinie et lorsque la période de temps prédéfinie a été atteinte, produire un signal de rétroaction de RCP conseillant à la personne d'arrêter la RCP.

11. Système d'évaluation de RCP (20) selon l'une quelconque des revendications précédentes, configuré pour répéter les étapes (i) à (viii).

12. Système d'évaluation de RCP (20) selon l'une quelconque des revendications précédentes, configuré pour exécuter les étapes :
production d'un signal de rétroaction de RCP conseillant à la personne d'effectuer des compressions thoraciques de RCP à une fréquence égale à une fréquence d'un signal de métronome audible émis par un métronome du système d'évaluation de RCP (20),
réception des signaux d'ECG mesurés pendant les compressions thoraciques et utilisation des signaux d'ECG pour établir une mesure de signal d'ECG courante,
comparaison de la mesure de signal d'ECG courante avec la mesure de signal d'ECG de référence,
lorsque la mesure de signal d'ECG courante est inférieure à la mesure de signal d'ECG de référence, ajustement de la fréquence du signal de métronome audible, production d'un signal de rétroaction de RCP conseillant à la personne d'ajuster la fréquence d'exécution des compressions thoraciques de RCP pour qu'elle soit égal à la fréquence ajustée du signal de métronome audible et retour à la deuxième étape,
lorsque la mesure de signal d'ECG courante est supérieure ou égale à la mesure de signal d'ECG de référence, production d'un signal de rétroaction de RCP conseillant à la personne de maintenir la fréquence d'exécution des compressions thoraciques de RCP.

13. Système de rétroaction de RCP (20) selon l'une quelconque des revendications précédentes qui fait partie d'un défibrillateur.
